# EUROPEAN PATENT SPECIFICATION

(11) **EP 4 061 226 B1**
(45) Date of publication and mention of the grant of the patent: **20.05.2026**
(21) Application number: 20808401.2
(22) Date of filing: 18.11.2020
(51) Int. Cl.: A61B 5/291, A61B 5/00

(54) **ELECTRODE FOR ELECTROENCEPHALOGRAPHY**
ELEKTRODE FÜR DIE ELEKTROENZEPHALOGRAFIE
ÉLECTRODE POUR ÉLECTRO-ENCÉPHALOGRAPHIE

(30) Priority: 20.11.2019 US 201962938061 P
(43) Date of publication of application: 28.09.2022
(73) Proprietor: Specs France SAS, 75008 Paris (FR)
(72) Inventor: RECHKE, Bastien, 75009 Paris (FR); PLOYART, Guillaume, 75009 Paris (FR); KOUIDER, Sid, 75009 Paris (FR)
(74) Representative: Kramer, Dani
(86) International application number: PCT/EP2020/082527
(87) International publication number: WO 2021/099382

(56) References cited:
- WO-A1-01/01856
- CN-A- 108 135 525
- JP-A- 2018 175 287
- US-A1- 2019 192 024

## Description

### Cross-Reference to Related Applications

This application claims the benefit of priority from U.S. Provisional Patent Application Serial Number 62/938,061, filed November 20, 2019, and entitled "ELECTRODE FOR ELECTROENCAPHALOGRAPHY".

### Technical field of the invention

Embodiments of the present disclosure relate to electrodes used in the acquisition of electroencephalographic (EEG) signals.

### State of the art

Surface electroencephalography makes it possible to measure the variations of diffuse electric potentials on the surface of the skull of a subject. These variations of electrical potentials are commonly referred to as electroencephalographic signals or EEG signals.

Extracting a reliable EEG signal, given the very small amplitude of the electrical potential variations to be measured (i.e. of the order of a few microvolts), drives development of surface EEG equipment that improves conductivity between sensors and the scalp. One obstacle to that improved contact can be condition (i.e. length, thickness and style) of the hair of the subject, which can significantly affect the impedance experienced by the measured electrical potentials.

To address the issue of signal reliability, some surface EEG devices are equipped with gel electrodes, in which contact is made through a gel or conductive liquid, which easily seeps through the user's hair to reach the scalp. The electrode itself is generally made of metal. The gel makes it possible to reduce the electrical impedance and thus the interference with surrounding signals without requiring physical contact between the electrode and the scalp. This solution provides good conductivity at any point of the scalp. Technical assistance is however required to ensure appropriate electrode placement, which in turn is time-consuming (since the gel must be applied and the conductance checked individually for each electrode).

In certain cases, the electrode includes circuitry (in addition to the metal "sensor" itself). This circuitry may process the analog signal coming from the sensor to amplify the electrical potentials captured at the sensor before that signal is routed to other electronic components. Such electrodes are termed the "active" wet electrodes. Amplification, in this context, means that a component strongly drives the voltage on a line to the level coming from the sensor. After amplification, the signal is less susceptible to interference as it is conveyed to the other components, before conversion (from analog to digital). The greater the impedance (due to hair condition, for example), the weaker the signal (in terms of driving voltage on the line) so this amplification circuitry can be essential for proper functioning even with gel.

The use of gel limits the duration of use of the device to a few hours (since the contact is no longer assured as the gel dries). In many cases, the conductive liquid or gel leaves a residue in the hair after the use of the EEG system, this can be difficult to remove.

More recently, surface electroencephalographs equipped with so-called "active dry" electrodes have been introduced: the electrodes being termed "dry" because they require no gel or other conductive liquid. Active dry electrodes operate by capturing the variations of electrical potential signals on the surface of the scalp, and then amplifying those signals (in some cases also filtering the signals). The analog signals thus obtained are then converted into digital signals by means of one or more analog-to-digital converters controlled by a microcontroller. The microcontroller receives the data for analysis, storage and/or onward transmission to another device.

In active dry electrodes, the contact with the scalp is through solid conducting elements or "sensors" connected to an electronic circuit to overcome the increase in impedance (compared to impedance in the presence of gel). The active dry electrode facilitates signal capture comparable to that of a gel electrode but also allows filtering and/or amplification of the captured signals, and thus an improved signal-to-noise ratio.

Stable access to the scalp limits the reliability of the signal capture. The shape of the sensors is restricted by the need for contact that extends through the hair of the subject.

It is known to provide pin-style active dry sensors (in a conductive polymer material) that require the application of significant pressure to reach the user's scalp. Such sensors are, due to the application of pressure at the interface of scalp and sensor pin, very uncomfortable, especially for prolonged use.

Known systems in the medical or related research fields generally include an unflattering cap, often of elastic or waterproof fabric, with attachment locations for receiving individual sensors/electrodes. Electronic circuits are then connected to the electrodes and to the housing of an acquisition chain (i.e. an assembly of connected components used in acquiring the EEG signals). The EEG device is thus typically formed of three distinct elements that the operator / exhibitor must assemble at each use. Again, the nature of the EEG device is such that technical assistance is desirable if not essential.

Furthermore, user acceptability of the EEG device (and its electrodes) places aesthetic constraints, as well as constraints in comfort and ease of use. In many cases, these constraints are an effective significant barrier to the adoption of EEG technology. Examples of applications where comfort over prolonged use and the need for technical assistance prevent adoption include applications such as video games, training (e.g. for health and safety or flight simulation), sleep aids, etc.

It is therefore desirable to provide electrodes for EEG devices that address the above challenges.

US 2019/0192024 Al describes a method for generating an EEG signal. Multiple signals from multiple electrodes applied to a user's scalp are measured. The signals correspond to electrical activity generated by the user's brain. Each of the signals is evaluated using a machine learning algorithm in real-time to determine a quality of each of the signals. One or more switches is activated between the electrodes to electrically-connect a plurality of the electrodes. An EEG signal is measured using the parallel-connected electrodes.

WO 2001/001856 Al describes a quick-placement EEG electrode. The EEG electrode is fixed to a patient's head by a first element, a second element or movable section that operates in conjunction with the fixed section to trap hair, and hold the EEG electrode in place. The EEG electrode contains a sponge that when compressed, dispenses electrolytic gel, acts as a shock absorber, and maintains scalp contact. The EEG electrode has a quick release mechanism with a break-away ring, and locking groove for easy removal of the EEG electrode from the patient's scalp.

### SUMMARY

The present disclosure relates to active dry electrodes that provide a balance of excellent signal quality, ease of use, comfort for the user and design freedom.

The invention and aspects of it can be summarized by the appended claims.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 illustrates an electronic architecture for receiving and processing EEG signals according to the present disclosure;
FIGS. 2A and 2B illustrate side and end views of an example of a sensor according to the present disclosure;
FIG. 3 illustrates a further view of an exemplary sensor within a resiliently deformable support mount assembly according to the present disclosure; and
FIG. 4 shows a schematic arrangement of an active dry electrode according to the present disclosure.

### DETAILED DESCRIPTION

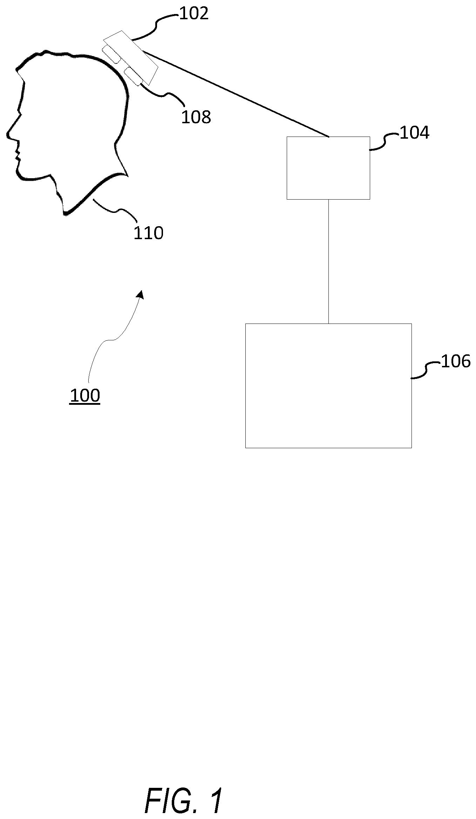

FIG. 1 illustrates an example of an electronic architecture for the reception and processing of EEG signals by means of an EEG device 100 according to the present disclosure.

To measure diffuse electric potentials on the surface of the skull of a subject 110, the EEG device 100 includes a portable device 102 (i.e. a cap or headpiece), analog-digital conversion (ADC) circuitry 104 and a microcontroller 106. The portable device 102 of FIG. 1 includes one or more active dry electrodes 108, typically between 1 and 128 electrodes, advantageously between 2 and 64, advantageously between 4 and 16.

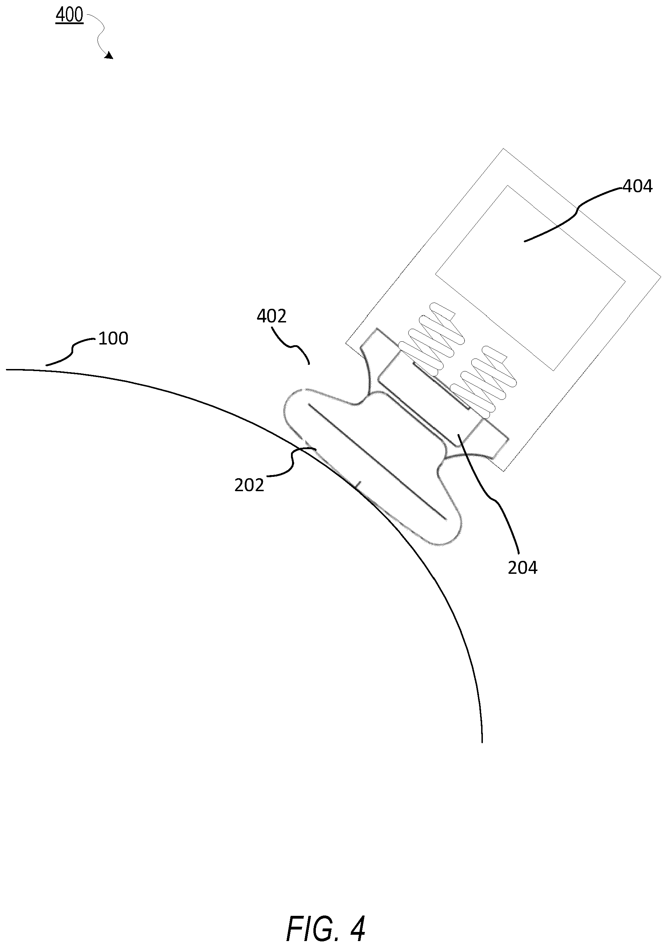

Each active electrode 108 comprises a sensor for detecting the electrical signals generated by the neuronal activity of the subject and an electronic filtering and amplifying circuit. These elements are discussed in more detail in relation to FIG. 4 below. The active electrodes 108 are shown in use in FIG. 1, where the sensor is in physical proximity with the subject's scalp.

Each ADC 104 is configured to convert the signals of a given number of active electrodes 108, for example between 1 and 128.

One or more of the active dry electrodes are configured as reference electrodes. The or each reference electrode is connected to all converters 104. The reference electrode or electrodes are preferably positioned in contact with the user's head in a region remote from that of the other active electrodes.

The converters 104 are controlled by the microcontroller 106 and communicate with it for example by the protocol SPI ("Serial Peripheral Interface"). The microcontroller 106 packages the received data for transmission to an external processing unit (not shown), for example a computer, a mobile phone, a virtual reality headset, an automotive or aeronautical computer system, for example a car computer or a computer system. airplane, for example by Bluetooth, Wi-Fi ("Wireless Fidelity") or Li-Fi ("Light Fidelity").

In certain embodiments, each active electrode 108 is powered by a battery (not shown in FIG. 1). The battery is conveniently provided in a housing of the portable device 102.

In certain embodiments, each active electrode 108 measures a respective electric potential value from which the potential measured by the reference electrode (Ei = Vi - Vref) is subtracted, and this difference value is digitized by means of the ADC 104 then transmitted by the microcontroller 106.

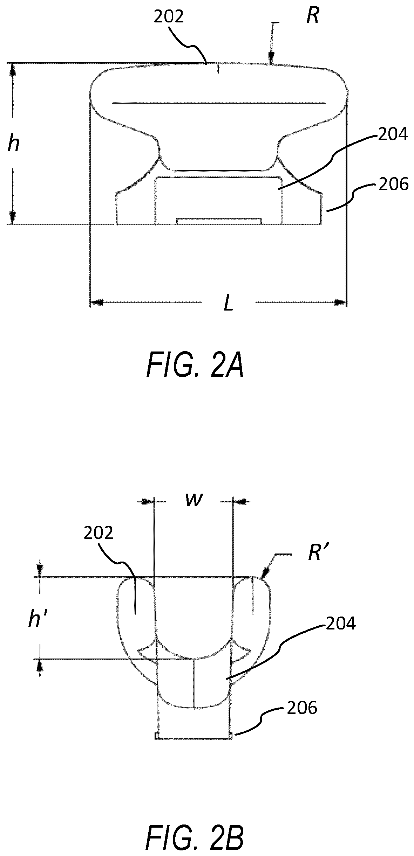

FIGs. 2A and 2B respectively illustrate a side view and a cross-section of a sensor (i.e. a solid conducting element of an active dry electrode) in accordance with the present disclosure.

The sensor is formed in an electrically conductive material (such as a conductive polymer). Conveniently, the sensor includes an embedded metal plate 204, the metal plate providing additional mechanical reinforcement. The sensor is shaped to include a curved contact surface 202: in use, the contact surface establishes a physical contact with the scalp of the subject 110.

As may be seen from the side views FIGs 2A, the contact surface has a curved profile, the curved profile being convex, bowing away from the body of the sensor with a radius of curvature, R (a typical value for R is of the order of tens of millimeters).

Fig. 2B represents a vertical cross-section through the center of the sensor of Fig. 2A. In accordance with the invention, as illustrated in FIGs 2A and 2B, the sensor has two parallel lobes (or "blades") each having the same convex curved profile. In cross-section, each blade is also rounded (with a typical radius of curvature R' of around a millimeter, say).

In accordance with the invention, as illustrated in FIGs. 2A and 2B, the blades are arranged substantially in parallel, so as to form contacts with the scalp of the subject along a substantially parallel lines when the device is worn. The conductive blades are formed of rigid material.

When brought into contact with the scalp, the contact surfaces 202 of each blade bow towards the head (i.e. they are convex in the direction locally normal to the surface of the scalp). This means that the curvature of each blade does not conform to the curvature of the scalp. While a concave blade shape would intuitively facilitate more effective contact along a greater portion of the linear contact surface, empirical study determined that the opposite was true. Two factors appear to determine how effective the contact may be established in reality: the combing effect and the blade tips.

While a concave blade shape provides tips at each end of the blade which might be imagined to help the sensor blade to part strands of hair, the convex form of blade is observed to part hair effectively with less resistance. Furthermore, the blade tips of a concave sensor blade appear to lift the blade from the scalp at points that are not at the tips, so that rather than distribute pressure along the blade more evenly (i.e. for better comfort) and maximizing the surface where the blade is in contact with the user's scalp (i.e. for better signals), the concave blade design actually performs worse for user comfort and signal reliability than a convex shape.

A further benefit of a convex sensor blade shape is that it is well-suited to injection molding production using well-known injection techniques (meaning that it is adapted to mass production), provided the sensor blades are formed of a suitably moldable material (in FIG. 1 the sensor blade is manufactured from a conductive polymer: one example being a thermoplastic charged with conductive particles, such as carbon material (fiber, powder or nanotubes)). No additional electrically conductive coating is needed where the whole sensor is made of a conductive polymer.

The sensor in FIGs. 2A and 2B is provided with a metal insert 204. A metal insert may not always be essential, depending upon the choice of material for the sensor blades and the degree of robustness required. Where present, the metal insert may serve a mechanical function reinforcing the material of the blade. The hardness of the conductive polymer may not be sufficient to provide a robust EEG device alone given the specific size constraints placed on the frame around the electrodes. If needed, the metal insert may also be used for its electrical properties to provide a conductive path from the user's scalp to the electronic circuit.

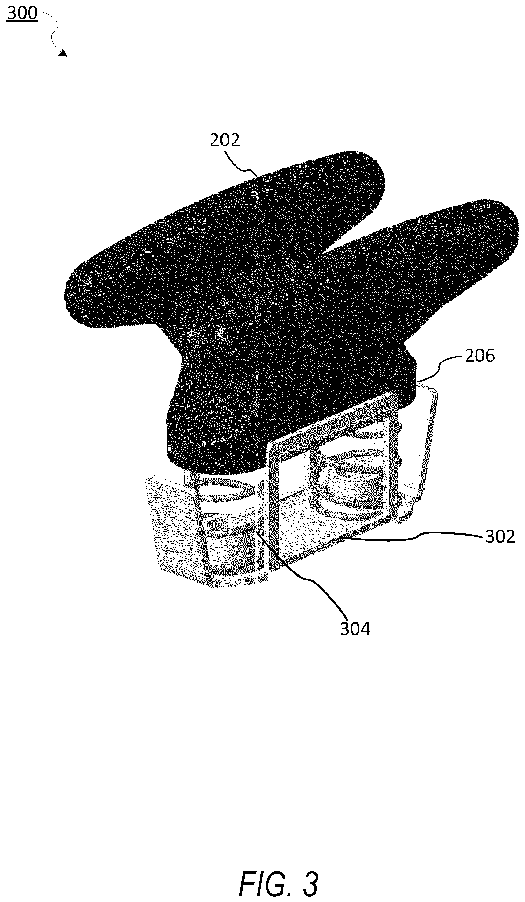

FIG. 3 shows a sensor mount arrangement 300 in which a sensor 200, such as that shown in FIGs. 2A and 2B, is mounted within a frame 302 (or cage). The sensor 200 is mounted on one or more coil springs 304 (here, two). The coil spring(s) 304 and a portion 206 of the sensor are restricted within the frame 302. A further portion of the sensor extends beyond the frame, the sensor blades being provided on the further portion. The coil spring 304 is compressed within the frame so that the sensor 200 is urged against a stop in the frame 302. The frame 302 (which may conveniently be made of metal) therefore fastens the sensor 200 inside the frame 302 while allowing the sensor 200 to retract, at least partially, into a recess within the frame when pressure is applied to the sensor blade (i.e. as the sensor blade is brought into contact with the surface of the subject's scalp).

Optionally, the frame 302 comprises an inner metal cage and an outer plastic frame thereby facilitating the assembly of the electrodes inside the frame. A subsystem comprising the sensor 200, the coil springs 304 and the metal cage can be preassembled and then clipped (e.g. snap-fitted) to the plastic frame. A spring finger may then be soldered onto electrical circuitry of an EEG device to make electrical contact with the metal cage.

In certain arrangements of electrodes for EEG devices, it may be convenient to provide electrodes serving as "reference" electrodes (which assist in providing a reference level of electrical activity against which measurement may occur). Conveniently, the electrode used as a reference electrode may take the same form as the measurement electrodes.

It is noted that the electrode shapes and dimensions described above are selected in order to accommodate the widest variety of hair types and lengths, and to facilitate setup and removal of the whole device, autonomously (i.e. by the subject themselves without additional technical assistance).

As discussed above, active dry electrodes make contact with the scalp of a subject through solid conducting elements or "sensors" connected to an electronic circuit. The sensors facilitate signal capture, while the corresponding electronic circuits allow amplification and/or filtering of the captured signals.

FIG. 4 illustrates an arrangement of functional elements of an active dry electrode 400 including a sensor 402, such as that illustrated in FIGs 2A, 2B and 3, and an electronic circuit 404. The active dry electrode may be used as one of the one or more active dry electrodes 108 in FIG. 1.

In certain embodiments, each electronic circuit 404 comprises a first-order highpass analog filter, an amplifier and a first-order low-pass analog filter. The filters make it possible to suppress signals that are detected by the frequency components that are useless for the intended application.

The active dry electrode 400 is shown in contact with the scalp of the subject 100. This may be at a localized region of the subject's head, for example, the occipital region at the rear of the skull.

The amplification at each electronic circuit 404 makes it possible to adapt the amplitude of the signals to the characteristics of the analog-digital converter (ADC) (104 in FIG. 1), and to obtain a maximum resolution during the conversion.

Although described through a number of detailed exemplary embodiments, the portable devices for the acquisition of electroencephalographic signals according to the present disclosure comprise various variants, modifications and improvements which will be obvious to those skilled in the art. The scope of the subject of the present invention is defined by the following claims.

Although an overview of the inventive subject matter has been described with reference to specific example embodiments, various modifications and changes may be made to these embodiments without departing from the broader scope of embodiments of the present disclosure. Such embodiments of the inventive subject matter may be referred to herein, individually or collectively, by the term "invention" merely for convenience and without intending to voluntarily limit the scope of this application to any single disclosure or inventive concept if more than one is, in fact, disclosed.

The embodiments illustrated herein are described in sufficient detail to enable those skilled in the art to practice the teachings disclosed. Other embodiments may be used and derived therefrom, such that structural and logical substitutions and changes may be made without departing from the scope of this disclosure. The Detailed Description, therefore, is not to be taken in a limiting sense, and the scope of various embodiments is defined only by the appended claims.

As used herein, the term "or" may be construed in either an inclusive or exclusive sense. Moreover, plural instances may be provided for resources, operations, or structures described herein as a single instance. Additionally, boundaries between various resources, operations, modules, engines, and data stores are somewhat arbitrary, and particular operations are illustrated in a context of specific illustrative configurations. Other allocations of functionality are envisioned and may fall within a scope of various embodiments of the present disclosure. In general, structures and functionality presented as separate resources in the example configurations may be implemented as a combined structure or resource. Similarly, structures and functionality presented as a single resource may be implemented as separate resources. These and other variations, modifications, additions, and improvements fall within a scope of embodiments of the present disclosure as represented by the appended claims. The specification and drawings are, accordingly, to be regarded in an illustrative rather than a restrictive sense.

## Claims

1. A sensor (200) for an electroencephalographic, EEG, device (100) for measuring electrical signals generated by the neuronal activity of a subject (110), the sensor (200) having two parallel blades formed of a rigid material and having an edge-to-edge spacing greater than 2 mm, each blade of the two parallel blades having the same curved convex profile and having a contact surface (202), the contact surface (202) having a curved convex profile such that the sensor (200) is arranged to be placed onto a scalp of the subject (110) with the curved convex profile being located in a plane transecting the scalp of the subject (110) and the curved convex profile being convex locally normal to the scalp of the subject (110),
wherein the curved convex profile of each blade of the sensor (200) forms a blade shape, the curvature of the blade shape having a curvature bowing away from a body of the sensor (200), wherein the sensor (200) is arranged in a frame (302), the frame (302), in use, urging the contact surfaces (202) of each blade of the sensor (200) towards the scalp so as to form contacts with the scalp when the device (100) is worn by the subject (110).

2. The sensor (200) of claim 1, wherein the sensor (200) is electrically coupled to an electronic circuit, the electronic circuit being configured to amplify the electrical signals.

3. The sensor (200) of claim 2, wherein the electronic circuit is further configured to operate to filter the amplified electrical signals.

4. The sensor (200) of claim 2 or 3, wherein the sensor (200) includes a connector for connecting to an electronic signal processing chain of the EEG device, the chain comprising one or more analog-to-digital converters, ADCs, (104) configured to transform the signals received from the electronic circuit into digital signals; and a microcontroller (106) for transmitting to an external processing unit and/or storing said digital signals.

5. An active dry electrode (108) including an electronic circuit and a sensor (200) as claimed in any one of claims 1-4, the electronic circuit being arranged to filter and amplify the electrical signals detected by the sensor (200).

6. An electroencephalographic, EEG, device (100) incorporating one or more active dry electrodes (108) as claimed in claim 5.

7. The EEG device (100) of claim 6, further including:
an electronic signal processing chain, the chain comprising one or more analog-to-digital converters, ADCs, (104) configured to transform the signals received from the electronic filtering and amplification circuits into digital signals; and
a microcontroller (106) for transmitting to an external processing unit and/or storing said digital signals.

## Patentansprüche

1. Sensor (200) für eine Elektroenzephalografievorrichtung, EEG-Vorrichtung, (100) zum Messen elektrischer Signale, die durch die neuronale Aktivität eines Probanden (110) erzeugt werden, wobei der Sensor (200) zwei parallele Flügel aufweist, die aus einem steifen Material hergestellt sind und einen Abstand von Kante zu Kante von mehr als 2 mm aufweist, wobei jeder Flügel der zwei parallelen Flügel dasselbe gewölbte konvexe Profil aufweist und eine Kontaktfläche (202) aufweist, wobei die Kontaktfläche (202) ein gewölbtes konvexes Profil aufweist, so dass der Sensor (200) dazu eingerichtet ist, auf einer Kopfhaut des Probanden (110) platziert zu werden, wobei sich das gewölbte konvexe Profil in einer Ebene befindet, die die Kopfhaut des Probanden (110) durchschneidet, und das gewölbte konvexe Profil lokal senkrecht zu der Kopfhaut des Probanden (110) konvex ist,
wobei das gewölbte konvexe Profil jedes Flügels des Sensors (200) eine Flügelform bildet, wobei die Wölbung der Flügelform eine Wölbung aufweist, die sich von einem Körper des Sensors (200) weg biegt, wobei der Sensor (200) in einem Gestell (302) eingerichtet ist, wobei das Gestell (302) im Gebrauch die Kontaktfläche (202) jedes Flügels des Sensors (200) zu der Kopfhaut hin treibt, um Kontakte mit der Kopfhaut zu bilden, wenn die Vorrichtung (100) von dem Probanden (110) getragen wird.

2. Sensor (200) nach Anspruch 1, wobei der Sensor (200) an eine elektronische Schaltung elektrisch gekoppelt ist, wobei die elektronische Schaltung dazu konfiguriert ist, die elektrischen Signale zu verstärken.

3. Sensor (200) nach Anspruch 2, wobei die elektronische Schaltung ferner dazu konfiguriert ist, dahingehend zu arbeiten, die verstärkten elektrischen Signale zu filtern.

4. Sensor (200) nach Anspruch 2 oder 3, wobei der Sensor (200) einen Anschluss zum Anschließen einer elektronischen Signalverarbeitungskette der EEG-Vorrichtung, wobei die Kette einen oder mehrere Analog-Digital-Umsetzer, ADU, (104) umfasst, die dazu konfiguriert sind, die von der elektronischen Schaltung empfangenen Signale in digitale Signale umzuwandeln; und einen Mikrocontroller (106) zum Übertragen der besagten digitalen Signale an eine externe Verarbeitungseinheit und/oder Speichern dieser Signale einschließt.

5. Aktive trockene Elektrode (108), einschließend eine elektronische Schaltung und einen Sensor (200) nach einem der Ansprüche 1-4, wobei die elektronische Schaltung dazu eingerichtet ist, die von dem Sensor (200) erfassten elektrischen Signale zu filtern und zu verstärken.

6. Elektroenzephalografievorrichtung, EEG-Vorrichtung, (100), die eine oder mehrere aktive trockene Elektroden (108) nach Anspruch 5 einbindet.

7. EEG-Vorrichtung (100) nach Anspruch 6, ferner einschließend:
eine elektronische Signalverarbeitungskette, wobei die Kette einen oder mehrere Analog-Digital-Umsetzer, ADU, (104) umfasst, die dazu konfiguriert sind, die von den elektronischen Filterungs- und Verstärkungsschaltungen empfangenen Signale in digitale Signale umzuwandeln; und
einen Mikrocontroller (106) zum Übertragen der besagten digitalen Signale an eine externe Verarbeitungseinheit und/oder Speichern dieser Signale.

## Revendications

1. Capteur (200) pour dispositif électroencéphalographique, EEG, (100) destiné à mesurer les signaux électriques générés par l'activité neuronale d'un sujet (110), le capteur (200) présentant deux lames parallèles formées d'un matériau rigide et présentant un espacement bord à bord supérieur à 2 mm, chaque lame des deux lames parallèles ayant le même profil convexe incurvé et ayant une surface de contact (202), la surface de contact (202) ayant un profil convexe incurvé de telle sorte que le capteur (200) soit conçu pour être placé sur le cuir chevelu du sujet (110), le profil convexe incurvé étant situé dans un plan coupant transversalement le cuir chevelu du sujet (110) et le profil convexe incurvé étant localement normal au cuir chevelu du sujet (110),
dans lequel le profil convexe incurvé de chaque lame du capteur (200) forme une forme de lame, la courbure de la forme de lame ayant une courbure s'éloignant d'un corps du capteur (200), dans lequel le capteur (200) est agencé dans un cadre (302), le cadre (302), durant l'utilisation, poussant les surfaces de contact (202) de chaque lame du capteur (200) vers le cuir chevelu de manière à former des contacts avec le cuir chevelu lorsque le dispositif (100) est porté par le sujet (110).

2. Capteur (200) selon la revendication 1, dans lequel le capteur (200) est couplé électriquement à un circuit électronique, le circuit électronique étant configuré pour amplifier les signaux électriques.

3. Capteur (200) selon la revendication 2, dans lequel le circuit électronique est configuré en outre pour fonctionner afin de filtrer les signaux électriques amplifiés.

4. Capteur (200) selon la revendication 2 ou 3, le capteur (200) comportant un connecteur pour une connexion à une chaîne de traitement de signal électronique du dispositif EEG, la chaîne comprenant un ou plusieurs convertisseurs analogiques-numériques, CAN, (104) configurés pour transformer les signaux reçus du circuit électronique en signaux numériques ; et un microcontrôleur (106) pour transmettre à une unité de traitement externe et/ou stocker lesdits signaux numériques.

5. Électrode sèche active (108) comportant un circuit électronique et un capteur (200) selon l'une quelconque des revendications 1 à 4, le circuit électronique étant conçu pour filtrer et amplifier les signaux électriques détectés par le capteur (200).

6. Dispositif électroencéphalographique, EEG, (100) incorporant une ou plusieurs électrodes sèches actives (108) selon la revendication 5.

7. Dispositif EEG (100) selon la revendication 6, comportant en outre :
une chaîne de traitement de signal électronique, la chaîne comprenant un ou plusieurs convertisseurs analogiques-numériques, CAN, (104) configurés pour transformer les signaux reçus des circuits de filtrage et d'amplification électroniques en signaux numériques ; et
un microcontrôleur (106) pour transmettre à une unité de traitement externe et/ou stocker lesdits signaux numériques.
